(19) Europäisches Patentamt
European Patent Office
Office européen des brevets

(11) **EP 3 636 393 B1**

(12) **EUROPEAN PATENT SPECIFICATION**

(45) Date of publication and mention
of the grant of the patent:
**31.05.2023 Bulletin 2023/22**

(21) Application number: **18814316.8**

(22) Date of filing: **08.06.2018**

(51) International Patent Classification (IPC):
**B25J 9/16** *(2006.01)*     **A61B 34/37** *(2016.01)*
**A61B 34/00** *(2016.01)*

(52) Cooperative Patent Classification (CPC):
**B25J 9/1689; A61B 34/37; A61B 34/76;**
**B25J 9/1676;** A61B 2034/105; A61B 2034/107;
A61B 2090/064; G05B 2219/36429;
G05B 2219/36432; G05B 2219/39339;
G05B 2219/45117

(86) International application number:
**PCT/CN2018/090441**

(87) International publication number:
**WO 2018/224038 (13.12.2018 Gazette 2018/50)**

(54) **MEDICAL ROBOT AND CONTROL METHOD THEREOF**

MEDIZINISCHER ROBOTER UND STEUERUNGSVERFAHREN DAFÜR

ROBOT MÉDICAL ET PROCÉDÉ DE COMMANDE ASSOCIÉ

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **09.06.2017 CN 201710432983**

(43) Date of publication of application:
**15.04.2020 Bulletin 2020/16**

(73) Proprietor: **Shanghai Microport Medbot (Group)**
**Co., Ltd.**
**Shanghai 201203 (CN)**

(72) Inventors:
 • **SHI, Yunlei**
  **Shanghai 201203 (CN)**
 • **HE, Chao**
  **Shanghai 201203 (CN)**
 • **WANG, Jiayin**
  **Shanghai 201203 (CN)**
 • **ZHU, Xiang**
  **Shanghai 201203 (CN)**
 • **YUAN, Shuai**
  **Shanghai 201203 (CN)**
 • **JIANG, Yizhi**
  **Shanghai 201203 (CN)**

(74) Representative: **Patentship**
**Patentanwaltsgesellschaft mbH**
**Elsenheimerstraße 65**
**80687 München (DE)**

(56) References cited:
WO-A2-02/060653          CN-A- 101 448 467
CN-A- 104 470 456        CN-A- 105 643 621
CN-A- 107 009 363        JP-A- 2011 254 975
KR-A- 20110 024 026

• HO S C ET AL: "ROBOT ASSISTED KNEE
SURGERY. ÖESTABLISHING A FORCE
CONTROL STRATEGY INCORPORATING
ACTIVE MOTION CONSTRAINT", IEEE
ENGINEERING IN MEDICINE AND BIOLOGY
MAGAZINE, IEEE SERVICE CENTER,
PISACATAWAY, NJ, US, vol. 14, no. 3, 1 May 1995
(1995-05-01), pages 292-300, XP000505085, ISSN:
0739-5175, DOI: 10.1109/51.391774

**Description**

TECHNICAL FIELD

The present disclosure relates to the field of medical equipment, and in particular, to a medical robot and a control method thereof.

BACKGROUND

Spinal surgery is a clinical discipline that deals with spine and spinal cord diseases mainly through surgical operation. The main operations of the spinal surgery include screw insertion and cutting. The key of screw insertion is the insertion point and insertion angle. Conventional manual screw insertion requires the operator to select the insertion point depending on operator's experience, and determine the insertion angle depending on the spatial sense and intraoperative fluoroscopy. And cutting includes osteotomy and resection of soft tissues. Undesired stimulations or cutting to the nerve during the cutting operation are extremely easy to cause irreversible injury to postoperative neurological function. The tools used in conventional cutting often are selected from osteotome, abrasion drill or bone rongeur. During cutting, the operation should be strictly restrained to a narrow space, So the safety of the operation depends on the spatial sense, sensitive hand feeling and fine operation ability of the operator. According to the statistics, it shows that the incidence of nerve injuries caused by the manual screw insertion of the operator is about 3-55%, and the incidence of nerve injuries during the cutting is 3.2-12.8%. It is the development direction of spinal surgery researches in the current precision medical requirement that how to improve the operation precision using a new technology and reduce the limitation of manual operation or the loss caused by human error.

In order to solve the precision problem of the positioning screw insertion operation, the SpineAssist system has been developed for spine positioning screw insertion, which has been uniquely approved by the FDA and CFDA in the world as the inventor knows. The key technology of this system is the precise position instruction control of parallel mechanical arms, and the safety strategy of this system is the navigation technology based on the registration of intraoperative fluoroscopy and preoperative CT. The system can significantly improve the efficiency and safety of the screw insertion operation with a precision error of less than 1 mm as well as reduce the fluoroscopic times. The SPINEBOT system developed by Hanyang University of Korea also aims at the positioning screw insertion operation. The control mode of this system adopts the position instruction control of serial mechanical arms, and the safety strategy of this system is the navigation technology based on the registration of infrared optical positioning and preoperative CT. The SPINEBOT system has a precision error of 1-2 mm. The spine screw insertion robot that also adopts the infrared navigation and the position instruction control of serial mechanical arms are the Neuroglide system and the TiRobot system of the Beijing Jishuitan Hospital.

The focus of the spinal surgical robots currently on the market is the positioning screw insertion operation, which mainly adopts the direct position instruction control of the robot joint or adopts the master-slave teleoperation control mode. Although these robots can achieve a high precision in the position information aspect, the usage scenarios are very limited, and the needs of robotic assistance for clinical cutting operations cannot be satisfied. In the cutting operation, the operator needs to sense the changes of the operating positions and forces in real time, and adjust the action at any time according to the changes. Although robot with the simple position instruction control and the master-slave control mode can achieve a higher position precision, the information exchange of the operating force of the operation is totally lost in actual use, which results in a complete dependency on the precision of the operator's operation. Moreover, the surgical system is subject to registration precision errors, especially the influence of the movement of the anatomical structure due to incomplete fixation, so that it's difficult to meet the safety and precision requirements of the cutting operation.

Chinese Patent Application No. CN104470456A discloses a surgical robot system, including: a surgical tool configured to perform surgery on each of a plurality of procedure regions in a surgical region which is divided into the plurality of procedure regions; a robot arm on which the surgical tool is mounted; a driver adjusting an operating speed at which the robot arm operates, based on a risk level which is set for a procedure region in which the surgical tool is disposed and configured to operate the robot arm; and a change unit configured to switch a surgical mode between an automatic mode, in which surgery is automatically performed on the procedure region, and a collaborative mode in which surgery is performed on the procedure region in collaboration with an operator, wherein the driver comprises: a driving instrument configured to, when the surgery mode is the automatic mode, adjust a driving speed for operating the robot arm; and a load instrument configured to, when the surgery mode is the collaborative mode, adjust a load which acts in a process where the operator operates the robot arm. The surgical robot system categorizes the procedure region based on the risk level and uses the driving instrument and the load instrument to adjust the speed of the surgical tool in regions of different risk levels, so as to reduce the risk of medical accidents. However, in the master-slave control mode, i.e., in the cooperation mode described in the patent, the surgical tool still operates depending on operator's operation, and just different loads are set for regions of different risk levels to change the sensitivity of the surgical tool in response to the operator's operation, which cannot change or resist any operation of the operator. Therefore, once a misoperation of the operator occurs, the surgical robot system is able to alleviate damages caused by the misoperation to a certain extent, and is impossible to fundamentally avoid the occurrence of such misoperations.

Ho et al., "ROBOT ASSISTED KNEE SURGERY. ESTABLISHING A FORCE CONTROL STRATEGY INCORPORATING ACTIVE MOTION CONSTRAINT", IEEE ENGINEERING IN MEDICINE AND BIOLOGY MAGAZINE, vol. 14, no. 3, 1 May 1995 (1995-05-01), pages 292-300, XP000505085, DOI: 10.1109/51.391774, ISSN: 0739-5175, discloses a medical robot as well as different force control strategies in which the robot motion is constrained.

WO 02/060653 A2 discloses an active-constraint robot. Therefore, there is a need for a medical robot that is able to correct the operator's operation, particularly in the master-slave control mode.

## SUMMARY

Some embodiments provide a medical robot and a control method thereof to improve the safety and precision of the operation of the medical robot.

The medical robot comprises:

an operator operating force acquisition unit, configured to acquire an operator operating force;
a robot operating force acquisition unit, configured to acquire a robot operating force according to the danger level of a region in which a robot tip is located;
a control module, configured to determine a desired velocity of the robot tip according to the operator operating force and the robot operating force, and convert velocity information into a control instruction;
a driving module; and
the robot tip;
wherein the driving module drives the robot tip to move according to the received control instruction.

Optionally, the operator operating force acquisition unit comprises:

a multi-dimensional force sensor, configured to acquire an input force of an operator; and
an operator operating force calculation module communicatively connected to the multi-dimensional force sensor and configured to convert the input force acquired by the multi-dimensional force sensor into the operator operating force in a Cartesian space according to a robot force mapping model.

Optionally, the operator operating force acquisition unit comprises:

a robot joint torque detection module, configured to detect a joint torque of the robot; and
an operator operating force calculation module communicatively connected to the robot joint torque detection module, the operator operating force calculation module decoupling the joint torque of the robot through dynamics to obtain the operator operating force.

Further, the medical robot further comprises a filtering module, configured to filter the operator operating force to filter out a jitter occurred during an operator operation.

According to the invention, the robot operating force acquisition unit comprises:

a medical object generation module, configured to establish a medical object model, and categorize the medical object model into different regions according to the danger level;
a coordinate matching module, configured to acquire coordinates of the robot tip, match the medical object model with the coordinates of the robot tip, and determine the region of the medical object model in which the robot tip is located;
a regional constraint strategy module, configured to generate different constraint strategies according to different danger levels of regions in which the robot tip may be located according to a safety restriction algorithm; and
a robot operating force calculation module, configured to combine the coordinate matching module and the regional constraint strategy module to obtain a corresponding robot operating force.

According to the invention, regions of the medical object model categorized according to different danger levels comprise a recommended region, a safe region, and a forbidden region.

The constraint strategy comprises:

not generating a robot operating force in the recommended region;
generating a robot operating force with the form of damping force opposite to the operator operating force in the safe region to reduce the velocity of the robot tip; and
generating a robot operating force away from the forbidden region in the forbidden region.

Further, the control module is configured to: obtain a desired velocity of the robot tip in the Cartesian space according to the operator operating force and the robot operating force;

convert the desired velocity of the robot tip in the Cartesian space to obtain a desired velocity of the robot tip in a robot joint space according to a Jacobian transformation; and
decouple the desired velocity of the robot tip in the robot joint space using a linear decoupling control algorithm to obtain a control torque of the robot joint to achieve the speed control of the robot.

Further, the formula for obtaining the desired velocity of the robot tip in the Cartesian space by the control module according to the operator operating force and the robot operating force is:

$$\dot{\vec{X}}_{des} = K_V(\vec{F_h} - \lambda \bullet \vec{F_b})$$

wherein $\vec{F_h}$ is the operator operating force, $\vec{F_b}$ is the robot operating force, $K_V$ is a proportional transformation coefficient of a force of the robot tip in the Cartesian space to a velocity of the robot tip in the Cartesian space, $\dot{\vec{X}}_{des}$ is the desired velocity of the robot tip in the Cartesian space, and $\lambda$ is a scaling factor of the robot operating force generated due to an excess of the limit.

Further, the formula for obtaining the desired velocity in the robot joint space by the control module according to the desired velocity of the robot tip in the Cartesian space is:

$$\dot{\theta}_d = J_{kins}^{-1} \bullet \dot{\vec{X}}_{des}$$

wherein $J_{kins}^{-1}$ is the inverse Jacobian matrix under the current medical robot configuration, and $\dot{\theta}_d$ is the desired velocity in the robot joint space.

Further, decoupling, by the control module, the desired velocity in the robot joint space using the linear decoupling control algorithm to obtain a control torque of the robot joint to achieve the speed control of the robot comprises:

establishing a complete robot control model;
categorizing the robot control model into a model-based control portion and a model-based servo deviation control portion; and
adding a torque generated by the model-based control portion to a torque generated by the model-based servo deviation control portion to obtain the control torque for the robot joint.

Further, the complete robot control model is:

$$\tau = M(\theta)\ddot{\theta} + V(\theta,\dot{\theta}) + G(\theta) + F(\theta,\dot{\theta})$$

wherein $M(\theta)$ is a model parameter of an inertia matrix of a robot operating arm, $V(\theta,\dot{\theta})$ is a model parameter of a velocity of a robot operating arm, $G(\theta)$ is a model parameter of a gravity of a robot operating arm, $F(\theta,\dot{\theta})$ is a model parameter of a friction of a robot operating arm, $\tau$ is a control torque of $n \times 1$ robot joints, $n$ is the degree of freedom of a robot operating arm joint, and $\theta$ is an actual angle in the robot joint space.

Further, the formula of the torque generated by the model-based control portion is:

$$\beta = V(\theta,\dot{\theta}) + G(\theta) + F(\theta,\dot{\theta})$$

the formula of the torque generated by the model-based servo deviation control portion is:

$$\tau' = \ddot{\theta}_d + k_v'\dot{E}$$

wherein $\theta_d$ is a desired angle in the robot joint space, $\ddot{\theta}_d$ is a desired acceleration in the robot joint space, $E = \theta_d - \theta$ is a deviation between the desired angle and the actual angle in the robot joint space, $\dot{E}$ is a deviation of the desired velocity in the robot joint space and the actual velocity in the robot joint space, and $k_v'$ is a gain factor of the speed deviation.

The other embodiments also provide a control method of a medical robot, comprising:

    acquiring an operator operating force;
    obtaining a robot operating force according to the danger level of a region in which a robot tip is located;
    determining a velocity of the robot tip according to the operator operating force and the robot operating force, and converting the velocity information into a control instruction; and driving the robot tip to move according to the control instruction.

Optionally, the step of acquiring an operator operating force comprises:

    acquiring, by the multi-dimensional force sensor located at the robot tip, an operator operating force in a multi-dimensional sensor coordinate system; and
    converting, by the operator operating force calculation module, the operator operating force in the multi-dimensional sensor coordinate system into the operator operating force in the Cartesian space according to the robot force control model.

Optionally, the step of acquiring an operator operating force comprises:

    detecting a joint torque of the robot; and
    decoupling the joint torque of the robot through dynamics to obtain the operator operating force.

Further, the step of obtaining a robot operating force according to the danger level of a region in which a robot tip is located comprises:

    establishing a medical object model;
    categorizing the medical object model into regions, and setting different robot operating forces for different regions;
    matching the medical object model with coordinates of the robot tip; and
    acquiring position coordinates of the robot tip, and determining a region of the medical object model in which the robot tip is located, to obtain the corresponding robot operating force.

Further, the step of determining a velocity of the robot tip according to the operator operating force and the robot operating force together comprises:

    obtaining a desired velocity of the robot tip in the Cartesian space according to the operator operating force and the robot operating force;
    converting the desired velocity of the robot tip in the Cartesian space to obtain a desired velocity in a robot joint space according to a Jacobian transformation; and
    decoupling the desired velocity in the robot joint space using the linear decoupling control algorithm to obtain a control torque of the robot joint.

Compared with the prior art, the embodiments have the following advantageous effects:
The medical robot and the control method thereof provided in embodiments determine the velocity of a robot tip according to an operator operating force and a robot operating force, so that a robot operating force is generated to keep the robot tip in the correct trajectory and region all the time when an operator operates the robot tip to deviate from a desired trajectory or enter a dangerous region. Therefore, the medical robot and the control method thereof are able to change the simple master-slave mode control of operator-robot in the prior art to enable information exchange between the operator and the robot, thereby greatly improving the safety and precision of medical robot operation.

BRIEF DESCRIPTION OF THE DRAWINGS

FIG. 1 is a schematic structural diagram of a medical robot according to some embodiments;
FIG. 2 is a schematic diagram of the region categorization in a medical object model of some embodiments;
FIG. 3 is a schematic diagram of the region categorization in another medical object model of some embodiments; and
FIG. 4 is a schematic diagram of an operating force strategy in another medical object model of some embodiments.

DETAILED DESCRIPTION

The medical robot and the control method thereof provided in the present disclosure are further described in detail below with reference to the accompanying drawings. Advantages and features of the present disclosure will be apparent from the description and appended claims below. It should be noted that the drawings are in a very simplified form and use non-precise proportions, with the only intention to facilitate convenience and clarity in explaining the object of the present disclosure.

The main concept of some embodiments is to provide a medical robot and a control method thereof, in which the velocity of a robot tip is determined by an operator operating force and a robot operating force together, so that the robot operating force is generated to keep the robot tip in the correct trajectory or region all the time when an operator operates the robot tip to deviate from a desired trajectory or enter a dangerous region. The medical robot and the control method thereof are able to change the simple master-slave mode control of operator-robot in the prior art to enable information exchange between the operator and the robot. So it greatly improves the safety and precision of medical robot operation. The term "velocity" used herein is a vector, including numerical value and direction.

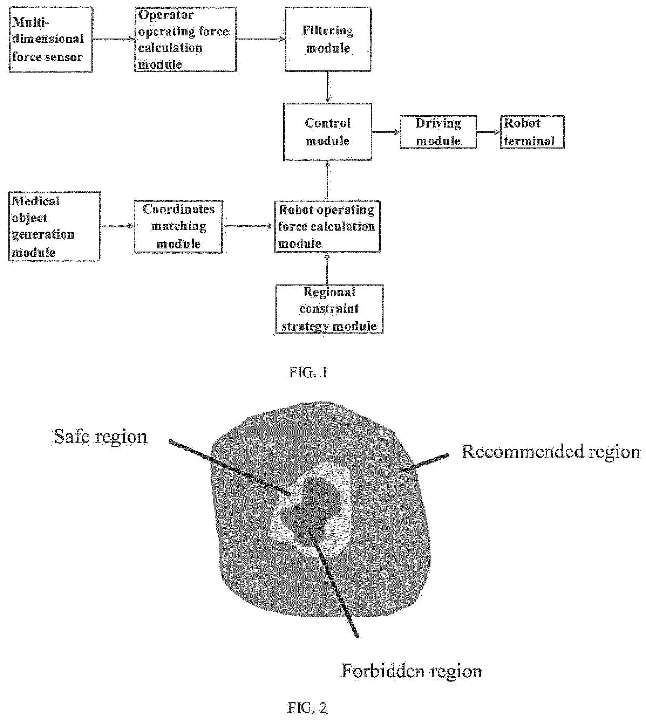

Referring to FIG. 1, which is a schematic structural diagram of a medical robot according to some embodiments.

As shown in FIG. 1, some embodiments provide a medical robot, comprising:

an operator operating force acquisition unit, configured to acquire an operator operating force;
a robot operating force acquisition unit, configured to acquire a robot operating force according to the danger level of a region in which a robot tip is located;
a control module, configured to determine a velocity of the robot tip according to the operator operating force and the robot operating force, and convert the velocity information into a control instruction;
a driving module, configured to receive the control instruction and drive the robot tip to move; and
the robot tip, driven by the driving module and configured to move according to the velocity.

As shown in FIG. 1, some embodiments also provide a control method of a medical robot, comprising:

acquiring an operator operating force;
obtaining a robot operating force according to the danger level of a region in which a robot tip is located;
determining a velocity of the robot tip according to the operator operating force and the robot operating force, and converting the velocity information into a control instruction; and driving the robot tip to move according to the control instruction.

The medical robot provided in some embodiments acquires the operator operating force and the robot operating force through the operator operating force acquisition unit and the robot operating force acquisition unit, respectively, and determines the velocity of the robot tip by the control module according to the two forces, so that a robot operating force is generated to keep the robot tip in the correct trajectory or region all the time or render the robot tip a tendency to move toward the desired region, when an operator operates the robot tip to deviate from a desired region. The medical robot and the control method thereof are able to change the simple master-slave mode control of the operator-robot in the prior art to enable information exchange between the operator and the robot, thereby greatly improving the safety and precision of medical robot operation.

In some embodiments, the operator operating force acquisition unit, the driving module, and the robot tip are disposed together to implement the control strategy of the medical robot. In other embodiments, the foregoing units and modules and the robot tip can also be disposed in a decentralized manner.

In some embodiments, the medical robot and the control method thereof are applied to a surgical operation to meet requirements of operating precision and safety required for surgery. It is conceivable that the medical robot and the control method thereof provided in some embodiments can also be applied to other fields requiring high precision manual operation.

In some embodiments, the operator operating force acquisition unit includes:

a multi-dimensional force sensor disposed at the robot tip. Preferably, a six-dimensional force sensor is adopted to obtain the operator operating force in a six-dimensional sensor coordinate system.

The multi-dimensional force sensor is communicatively connected to an operator operating force calculation module.

The operator operating force calculation module converts an operator operating force in the multi-dimensional sensor coordinate system into an operator operating force in the Cartesian space according to a robot force control model.

The step of acquiring the operator operating force by the operator operating force acquisition unit includes:

acquiring, by a multi-dimensional force sensor disposed at the robot tip, an operator operating force in a multi-dimensional sensor coordinate system; and

converting, by the operator operating force calculation module according to a robot force control model, the operator operating force in the multi-dimensional sensor coordinate system into the operator operating force in the Cartesian space.

It is conceivable that another optional operator operating force acquisition unit includes:

a robot j oint torque detection module, configured to detect a j oint torque of the robot; and

an operator operating force calculation module communicatively connected to the robot joint torque detection module.

The operator operating force calculation module decouples the joint torque of the robot through dynamics to obtain the operator operating force.

Specifically, the robot joint torque detection module may be an electric current detection module, or may be other detection modules for detecting the joint torque of the robot, such as a mechanical module for torque detection. Therefore, these technical solutions are included within the scope of the disclosure.

Accordingly, the step of acquiring the operator operating force by another operator operating force acquisition unit includes:

detecting a joint torque of the robot; and

decoupling the joint torque of the robot through dynamics to obtain the operator operating force.

The method for detecting the joint torque of the robot may be based on an electric current detection method, or other methods that are able to detect the joint torque of the robot. And the technical solutions used for the detection of the joint torque of the robot are all included within the scope of the disclosure.

Further, the medical robot further comprises a filtering module, configured to filter the operator operating force to filter out a jitter occurred during an operator operation.

It is able to effectively filter out the hand shake during an operator operation to perform a filtering on the acquired operator operating force. The filtering operation which combines with the robot operating force to jointly determine the velocity of the robot tip, will effectively improves the operation precision and efficiency of the robot tip. The filter processing mode may be a low-pass filtering or other filtering processing mode. And the technical solutions used for filtering out the jitter of the operator operation are all included within the scope of the disclosure.

It is obvious that the filtering module can be a low-pass filtering module or other filtering modules. And, the filtering modules used for filtering out the jitter of the operator operation are all included within the scope of the disclosure.

In some embodiments, the robot operating force acquisition unit comprises:

a medical object generation module, configured to establish a medical object model, and the medical object model is categorized into different regions according to the danger level;

a coordinate matching module, configured to acquire coordinates of the robot tip, match the medical object model with the coordinates of the robot tip, and determine the region of the medical object model in which the robot tip is located;

a regional constraint strategy module, configured to generate different constraint strategies according to different danger levels of regions in which the robot tip may be located according to a safety restriction algorithm; and

a robot operating force calculation module, configured to combine the coordinate matching module and the regional constraint strategy module to obtain a corresponding robot operating force.

Further, the step of obtaining a robot operating force by the robot operating force acquisition unit according to the region in which the robot tip is located includes:

establishing a medical object model;

categorizing the medical object model into regions, and setting different robot operating forces for different regions;
matching the medical object model with coordinates of the robot tip; and
acquiring position coordinates of the robot tip, and determining a region of the medical object model in which the robot tip is located to obtain a corresponding robot operating force.

Specifically, the step of categorizing regions of the medical object model includes:
categorizing the medical object model into a recommended region, a safe region, and a forbidden region.

For example, according to a medical image acquired by scanning of a CT machine or MRI equipment, the nuclear magnetic imaging technology is adopted to establish two-dimensional or three-dimensional medical models. The operation path is established and the dangerous region is determined, according to the operator's experience and viscera positions. Then, different force feedback parameters are set according to the different operations and different dangerous regions.

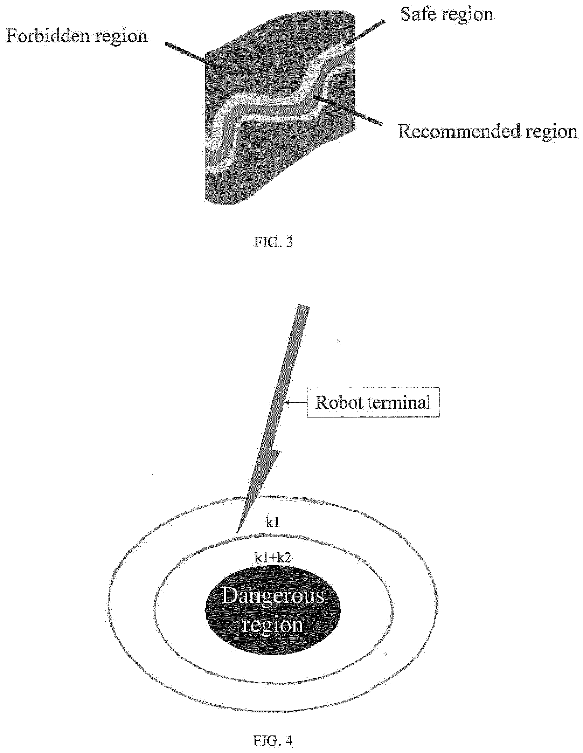

FIG. 2 and FIG. 3 illustrate two exemplary examples of the region categorization of the medical object model. The operation path and the dangerous region are as shown in FIG. 2 and FIG. 3 Based on a lesion image and the robot configuration, for different surgical tasks such as soft tissue resection and osteotomy, the constrained boundary is determined by combining the matched medical image with the posture and contour of the tool. Further, the robot is ensured to operate in a safe region by the regional constraint strategy module with the safety restriction algorithm. In FIG. 2, the forbidden region is surrounded by the recommended region, and the purpose of the safety restriction algorithm is to allow the operator to operate as far as possible from the forbidden region while in the recommended region. In FIG. 3, the recommended region is surrounded by the forbidden region. In this case, the regional constraint strategy module generally establishes a specified path and allows the operator to operate in the recommended region where the specified path is disposed as much as possible, so as to operate away from the forbidden region.

It is obvious that the region of the medical object model can also be classified in other ways. For example, the recommended region is isolated from the forbidden region instead of the surrounding relation. And, the divisional methods used for categorizing the region which make the robot tip is always in the desired region or has a tendency to move towards the desired region are all included within the scope of the disclosure.

The regional constraint strategy module does not generate additional robot operating force in the "recommended region", generates the robot operating force with the form of the damping force in the "safe region" to reduce the operator's operating speed, and generates the robot operating force away from the "forbidden region" in the "forbidden region", so as to assist the operator in performing safe and reasonable operations.

Further, the step of setting different robot operating forces for different regions specifically includes:

setting not to generate the robot operating force in the recommended region;
setting to generate a robot operating force with the form of the damping force opposite to the operator operating force in the safe region to reduce the velocity of the robot tip; and
setting to generate the robot operating force away from the forbidden region in the forbidden region.

It is obvious that the robot operating force can also be set by the other methods. For example, the regions of the medical object model categorized according to different danger levels comprise a dangerous region and a non-dangerous region. The boundary of the dangerous region is set according to the medical image. Further, the different proportional parameters of force feedback are set according to the distance between the robot tip and the boundary of the dangerous region. And then, the different force feedback prompts are sent to surgeons (i.e., operators). Finally, the hierarchical force feedback strategy is realized. In the example shown in FIG. 4, the proportional parameter of the force feedback is k1 when the robot tip is away from the dangerous region of the surgical operation, and the proportional parameter of the force feedback is k1+k2 when the robot tip approaches the dangerous region of the surgical operation, where k1 and k2 are greater than zero.

Further, the step of acquiring a robot operating force by the robot operating force acquisition unit according to the danger level of a region in which a robot tip is located can include:
generating a desired trajectory of the robot tip on a display device such as a screen according to the matching information of the coordinates of the robot tip and the medical object model, so as to guide the operator to operate the robot tip in the desired trajectory.

Further, the step of determining a velocity of the robot tip by the control module according to the operator operating force and the robot operating force includes:

obtaining a desired velocity of the robot tip in the Cartesian space according to the operator operating force and the robot operating force;
converting the desired velocity of the robot tip in the Cartesian space to obtain a desired velocity in the robot joint space according to a Jacobian transformation; and

decoupling the desired velocity in the robot joint space using a linear decoupling control algorithm to obtain a control torque of the robot joint to achieve the speed control of the robot.

Further, kinematics analysis is performed based on the medical robot configuration, to obtain the transformation of the movement between the robot joint space and the Cartesian space. For example, a velocity Jacobian mapping model of the robot is obtained by a vector method. A force Jacobian mapping model is obtained by transforming the velocity Jacobian model. According to the foregoing models, the transformation between the robot task space and the robot joint space can be obtained.

Further, the formula for the desired velocity of the robot tip in the Cartesian space is:

$$\dot{\vec{X}}_{des} = K_V(\vec{F_h} - \lambda \bullet \vec{F_b})$$

wherein $\vec{F_h}$ is the operator operating force, $\vec{F_b}$ is the robot operating force, $K_V$ is a proportional transformation coefficient of the force of the robot tip in the Cartesian space to the velocity of the robot tip in the Cartesian space, $\lambda$ is a scaling factor of the robot operating force generated due to an excess of the limit, and $\dot{\vec{X}}_{des}$ is the desired velocity of the robot tip in the Cartesian space.

Further, the formula for the desired velocity in the robot joint space is:

$$\dot{\theta}_d = J_{kins}^{-1} \bullet \dot{\vec{X}}_{des}$$

wherein $J_{kins}^{-1}$ is the inverse Jacobian matrix under the current medical robot configuration, and $\dot{\theta}_d$ is the desired velocity in the robot joint space.

Further, decoupling, by the control module, the desired velocity in the robot joint space using the linear decoupling control algorithm to obtain a control torque of the robot joint to achieve the speed control of the robot includes:

establishing a complete robot control model;
categorizing the robot control model into a model-based control portion and a model-based servo deviation control portion; and
adding a torque generated by the model-based control portion to a torque generated by the model-based servo deviation control portion to obtain the control torque for the robot joint.

Further, the complete robot control model is:

$$\tau = M(\theta)\ddot{\theta} + V(\theta,\dot{\theta}) + G(\theta) + F(\theta,\dot{\theta})$$

wherein $M(\theta)$ is a model parameter of an inertia matrix of the robot, $V(\theta,\dot{\theta})$ is a model parameter of a velocity term, $G(\theta)$ is a model parameter of a gravity term, $F(\theta,\dot{\theta})$ is a model parameter of a friction term, $\tau$ is a control torque of $n \times 1$ robot joints, $n$ is the degree of freedom of the robot joint, and $\theta$ is an actual angle in the robot joint space.

Further, the formulas for the model-based control portion and servo deviation control portion are:

$$\tau = \alpha\tau' + \beta$$

$$\alpha = M(\theta)$$

$$\beta = V(\theta,\dot{\theta}) + G(\theta) + F(\theta,\dot{\theta})$$

$$\tau' = \ddot{\theta}_d + k_v' E$$

$$E = \theta_d - \theta$$

wherein $\theta_d$ is a desired angle in the robot joint space, $\ddot{\theta}_d$ is a desired acceleration in the robot joint space, $E$ is a deviation between the desired angle and the actual angle in the robot joint space, $\dot{E}$ is a deviation of the desired velocity in the robot joint space and the actual velocity in the joint space, $\tau'$ is a torque generated by the servo deviation control portion, $\beta$ is a compensation torque generated by the model-based portion, and $k_v'$ is a gain factor of the velocity deviation.

According to the control method of the medical robot provided in some embodiments determines the velocity of a robot tip through an operator operating force and a robot operating force. And, a robot operating force is generated to keep the robot tip in the correct trajectory or region all the time when an operator operates the robot tip to deviate from a desired trajectory or enter a dangerous region. The medical robot and the control method thereof are able to change the simple master-slave mode control of the operator-robot in the prior art to enable information exchange between the operator and the robot, thereby greatly improving the safety and precision of medical robot operation.

**Claims**

1. A medical robot, comprising:

    an operator operating force acquisition unit, configured to acquire an operator operating force;
    a robot operating force acquisition unit, configured to acquire a robot operating force according to a danger level of a region in which a robot tip is located, and comprising: a medical object generation module configured to establish a medical object model and categorizing the medical object model into different regions according to the danger level; a coordinate matching module, configured to acquire coordinates of the robot tip, matching the medical object model with the coordinates of the robot tip, and determining the region of the medical object model in which the robot tip is located; a regional constraint strategy module, configured to generate different constraint strategies according to different danger levels of regions in which the robot tip may be located according to a safety restriction algorithm; and a robot operating force calculation module, configured to combine the coordinate matching module and the regional constraint strategy module to obtain a corresponding robot operating force;
    a control module, configured to determine a desired velocity of the robot tip according to the operator operating force and the robot operating force, and convert velocity information into a control instruction;
    a driving module; and
    the robot tip;
    wherein the driving module is configured to drive the robot tip to move according to a received control instruction, **characterised in that**
    regions of the medical object model categorized according to different danger levels comprise a recommended region, a safe region, and a forbidden region;
    also **characterised in that** the constraint strategy comprises:

        not generating a robot operating force in the recommended region;
        generating a robot operating force with the form of damping force opposite to the operator operating force in the safe region to reduce a velocity of the robot tip; and

    generating a robot operating force away from the forbidden region in the forbidden region.

2. The medical robot according to claim 1, wherein the operator operating force acquisition unit comprises:

    a multi-dimensional force sensor, configured to acquire an input force of an operator; and
    an operator operating force calculation module communicatively connected to the multi-dimensional force sensor and configured to convert the input force acquired by the multi-dimensional force sensor into the operator operating force in a Cartesian space according to a robot force mapping model, or
    the operator operating force acquisition unit comprises:

        a robot joint torque detection module, configured to detect a joint torque of the robot; and

an operator operating force calculation module communicatively connected to the robot joint torque detection module, the operator operating force calculation module decoupling the joint torque of the robot through dynamics to obtain the operator operating force.

3. The medical robot according to claim 1 or 2, further comprising a filtering module, configured to filter the operator operating force to filter out a jitter occurred during an operator operation.

4. The medical robot according to any one of claims 1 to 3, wherein the control module is configured to: obtain a desired velocity of the robot tip in the Cartesian space according to the operator operating force and the robot operating force;

   convert the desired velocity of the robot tip in the Cartesian space to a desired velocity of the robot tip in a robot joint space according to a Jacobian transformation; and
   decouple the desired velocity of the robot tip in the robot joint space using a linear decoupling control algorithm to obtain a control torque of a robot joint to achieve a speed control of the robot.

5. The medical robot according to claim 4, wherein a formula for obtaining the desired velocity of the robot tip in the Cartesian space by the control module according to the operator operating force and the robot operating force is:

$$\dot{\vec{X}}_{des} = K_V (\vec{F}_h - \lambda \bullet \vec{F}_b)$$

wherein $\vec{F}_h$ is the operator operating force, $\vec{F}_b$ is the robot operating force, $K_V$ is a proportional transformation coefficient of a force of the robot tip in the Cartesian space to a velocity of the robot tip in the Cartesian space, $\dot{\vec{X}}_{des}$ is the desired velocity of the robot tip in the Cartesian space, and $\lambda$ is a scaling factor of the robot operating force generated due to an excess of a limit.

6. The medical robot according to claim 4, wherein a formula for obtaining the desired velocity in the robot joint space by the control module according to the desired velocity of the robot tip in the Cartesian space is:

$$\dot{\theta}_d = J_{kins}^{-1} \bullet \dot{\vec{X}}_{des}$$

wherein $J_{kins}^{-1}$ is an inverse Jacobian matrix under the current medical robot configuration, and $\dot{\theta}_d$ is the desired velocity in the robot joint space.

7. The medical robot according to claim 4, wherein decoupling, by the control module, the desired velocity in the robot joint space using a linear decoupling control algorithm to obtain the control torque of the robot joint to achieve the speed control of the robot comprises:

   establishing a complete robot control model;
   categorizing the robot control model into a model-based control portion and a model-based servo deviation control portion; and
   adding a torque generated by the model-based control portion to a torque generated by the model-based servo deviation control portion to obtain the control torque for the robot joint.

8. The medical robot of claim 7, wherein the complete robot control model is:

$$\tau = M(\theta)\ddot{\theta} + V(\theta,\dot{\theta}) + G(\theta) + F(\theta,\dot{\theta})$$

wherein $M(\theta)$ is a model parameter of an inertia matrix of a robot operating arm, $V(\theta,\dot{\theta})$ is a model parameter of a velocity of a robot operating arm, $G(\theta)$ is a model parameter of a gravity of a robot operating arm, $F(\theta,\dot{\theta})$ is a model parameter of a friction of a robot operating arm, $\tau$ is a control torque of $n \times 1$ robot joints, $n$ is a degree of freedom of a robot operating arm joint, and $\theta$ is an actual angle in the robot joint space, and wherein

the formula of the torque generated by the model-based control portion is:

$$\beta = V(\theta,\dot{\theta}) + G(\theta) + F(\theta,\dot{\theta})$$

the formula of the torque generated by the model-based servo deviation control portion is:

$$\tau' = \ddot{\theta}_d + k_v' \dot{E}$$

wherein $\theta_d$ is a desired angle in the robot joint space, $\ddot{\theta}_d$ is a desired acceleration in the robot joint space, $E = \theta_d - \theta$ is a deviation between the desired angle and the actual angle in the robot joint space, $\dot{E}$ is a deviation of the desired velocity in the robot joint space and the actual velocity in the robot joint space, and $k_v'$ is a gain factor of the speed deviation, $\tau'$ is a torque generated by the servo deviation control portion, $\beta$ is a compensation torque generated by the model-based portion.

9. The medical robot of claim 1, wherein the forbidden region is surrounded by the recommended region.

10. A control method of a medical robot as in claim 1, comprising:

acquiring an operator operating force;
obtaining a robot operating force according to a danger level of a region in which a robot tip is located and according to the constraint strategy of claim 1, whereby the regions are defined as in claim 1;
determining a desired velocity of the robot tip according to the operator operating force and the robot operating force, and converting information of the velocity into a control instruction; and
driving the robot tip to move according to the control instruction.

11. The control method according to claim 10, wherein

the step of acquiring an operator operating force comprises:

acquiring, by a multi-dimensional force sensor disposed at the robot tip, an operator operating force in a multi-dimensional sensor coordinate system ; and
converting, by an operator operating force calculation module according to a robot force control model, the operator operating force in the multi-dimensional sensor coordinate system into an operator operating force in the Cartesian space , or

the step of acquiring an operator operating force comprises:

detecting a joint torque of the robot; and
decoupling the joint torque of the robot through dynamics to obtain the operator operating force.

12. The control method according to claim 10, wherein
the step of obtaining a robot operating force according to the danger level of a region in which a robot tip is located comprises:

establishing a medical object model;
categorizing the medical object model into regions, and setting different robot operating forces for different regions;
matching the medical object model with coordinates of the robot tip; and
acquiring position coordinates of the robot tip, and determining the region of the medical object model in which the robot tip is located, to obtain a corresponding robot operating force.

13. The control method according to claim 10, wherein

the step of determining a velocity of the robot tip according to the operator operating force and the robot operating force together comprises:

obtaining a desired velocity of the robot tip in the Cartesian space according to the operator operating force and the robot operating force;
converting the desired velocity of the robot tip in the Cartesian space to obtain a desired velocity in a robot joint space according to a Jacobian transformation; and
decoupling the desired velocity in the robot joint space using the linear decoupling control algorithm to obtain a control torque of a robot joint.

**Patentansprüche**

1. Medizinischer Roboter, umfassend:

eine Bedienerbedienkrafterfassungseinheit, die ausgebildet ist, eine Bedienerbedienkraft zu erfassen;
eine Roboterbedienkrafterfassungseinheit, die ausgebildet ist, eine Roboterbedienkraft gemäß einem Gefahrenniveau einer Region, in der sich eine Roboterspitze befindet, zu erfassen und umfassend: ein medizinisches Objekterzeugungsmodul, das ausgebildet ist, ein medizinisches Objektmodell zu erstellen und das medizinische Objektmodell gemäß dem Gefahrenniveau in verschiedene Regionen zu kategorisieren; ein Koordinatenanpassungsmodul, das ausgebildet ist, Koordinaten der Roboterspitze zu erfassen, das medizinische Objektmodell an die Koordinaten der Roboterspitze anzupassen und die Region des medizinischen Objektmodells zu bestimmen, in dem sich die Roboterspitze befindet; ein Regionalbeschränkungsstrategiemodul, das ausgebildet ist, gemäß einem Sicherheitsbeschränkungsalgorithmus unterschiedliche Beschränkungsstrategien gemäß unterschiedlichen Gefahrenniveaus von Regionen zu erzeugen, in denen sich die Roboterspitze befinden kann; und ein Roboterbedienkraftberechnungsmodul, das ausgebildet ist, das Koordinatenanpassungsmodul und das Regionalbeschränkungsstrategiemodul zu kombinieren, um eine entsprechende Roboterbedienkraft zu erhalten;
ein Steuerungsmodul, das ausgebildet ist, eine gewünschte Geschwindigkeit der Roboterspitze gemäß der Bedienerbedienkraft und der Roboterbedienkraft zu bestimmen, und Geschwindigkeitsinformationen in eine Steuerungsanweisung umzuwandeln;
ein Antriebsmodul; und
die Roboterspitze;
wobei das Antriebsmodul ausgebildet ist, die Roboterspitze anzutreiben, sich gemäß einer empfangenen Steuerungsanweisung zu bewegen,
**dadurch gekennzeichnet, dass** Regionen des medizinischen Objektmodells, die gemäß verschiedenen Gefahrenniveaus kategorisiert sind, eine empfohlene Region, eine sichere Region und eine verbotene Region umfassen;
ferner **dadurch gekennzeichnet, dass** die Beschränkungsstrategie umfasst:

eine Roboterbedienkraft in der empfohlenen Region nicht zu erzeugen;
eine Roboterbedienkraft mit der Form einer Dämpfungskraft entgegengesetzt zu der Bedienerbedienkraft in der sicheren Region zu erzeugen, um eine Geschwindigkeit der Roboterspitze zu reduzieren; und
eine Roboterbedienkraft weg von der verbotenen Region in der verbotenen Region zu erzeugen.

2. Medizinischer Roboter nach Anspruch 1, wobei die Bedienerbedienkrafterfassungseinheit umfasst:

einen mehrdimensionalen Kraftsensor, der ausgebildet ist, eine Eingabekraft eines Bedieners zu erfassen; und
ein Bedienerbedienkraftberechnungsmodul, das mit dem mehrdimensionalen Kraftsensor kommunikativ verbunden und ausgebildet ist, die durch den mehrdimensionalen Kraftsensor erfasste Eingabekraft in die Bedienerbedienkraft in einem kartesischen Raum gemäß einem Roboterkraftabbildungsmodell umzuwandeln, oder wobei die Bedienerbedienkrafterfassungseinheit umfasst:

ein Robotergelenkdrehmomentdetektionsmodul, das ausgebildet ist, ein Gelenkdrehmoment des Roboters zu detektieren; und
ein Bedienerbedienkraftberechnungsmodul, das mit dem Robotergelenkdrehmomentdetektionsmodul kommunikativ verbunden ist, wobei das Bedienerbedienkraftberechnungsmodul das Gelenkdrehmoment des Roboters durch Dynamik entkoppelt, um die Bedienerbedienkraft zu erhalten.

3. Medizinischer Roboter nach Anspruch 1 oder 2, ferner umfassend ein Filtermodul, das ausgebildet ist, die Bedienerbedienkraft zu filtern, um einen Jitter herauszufiltern, der während einer Bedienerbedienung aufgetreten ist.

4. Medizinischer Roboter nach einem der Ansprüche 1 bis 3, wobei das Steuerungsmodul ausgebildet ist: eine gewünschte Geschwindigkeit der Roboterspitze in dem kartesischen Raum gemäß der Bedienerbedienkraft und der Roboterbedienkraft zu erhalten;

die gewünschte Geschwindigkeit der Roboterspitze in dem kartesischen Raum in eine gewünschte Geschwindigkeit der Roboterspitze in einem Robotergelenkraum gemäß einer Jacobi-Transformation umzuwandeln; und die gewünschte Geschwindigkeit der Roboterspitze in dem Robotergelenkraum unter Verwendung eines linearen Entkopplungssteuerungsalgorithmus zu entkoppeln, um ein Steuerungsdrehmoment eines Robotergelenks zu erhalten, um eine Geschwindigkeitssteuerung des Roboters zu erreichen.

5. Medizinischer Roboter nach Anspruch 4, wobei eine Formel zum Erhalten der gewünschten Geschwindigkeit der Roboterspitze in dem kartesischen Raum durch das Steuerungsmodul gemäß der Bedienerbedienkraft und der Roboterbedienkraft ist:

$$\overrightarrow{\dot{X}}_{des} = K_V (\overrightarrow{\dot{F}_h} - \lambda \bullet \overrightarrow{\dot{F}_b})$$

wobei $\overrightarrow{F_h}$ die Bedienerbedienkraft ist, $\overrightarrow{F_b}$ die Roboterbedienkraft ist, $K_V$ ein proportionaler Transformationskoeffizient einer Kraft der Roboterspitze in dem kartesischen Raum zu einer Geschwindigkeit der Roboterspitze in dem kartesischen Raum ist, $\overrightarrow{\dot{X}}_{des}$ die gewünschte Geschwindigkeit der Roboterspitze in dem kartesischen Raum ist, und $\lambda$ ein Skalierungsfaktor der Roboterbedienkraft ist, die aufgrund einer Überschreitung einer Grenze erzeugt wird.

6. Medizinischer Roboter nach Anspruch 4, wobei eine Formel zum Erhalten der gewünschten Geschwindigkeit in dem Robotergelenkraum durch das Steuerungsmodul gemäß der gewünschten Geschwindigkeit der Roboterspitze in dem kartesischen Raum ist:

$$\dot{\theta}_d = J_{kins}^{-1} \bullet \overrightarrow{\dot{X}}_{des}$$

wobei $J_{kins}^{-1}$ eine inverse Jacobi-Matrix unter der aktuellen Konfiguration des medizinischen Roboters ist, und $\dot{\theta}_d$ die gewünschte Geschwindigkeit in dem Robotergelenkraum ist.

7. Medizinischer Roboter nach Anspruch 4, wobei das Entkoppeln, durch das Steuerungsmodul, der gewünschten Geschwindigkeit der Roboterspitze in dem Robotergelenkraum unter Verwendung eines linearen Entkopplungssteuerungsalgorithmus, um das Steuerungsdrehmoment des Robotergelenks zu erhalten, um die Geschwindigkeitssteuerung des Roboters zu erreichen, umfasst:

Erstellen eines vollständigen Robotersteuerungsmodells;
Kategorisieren des Robotersteuerungsmodells in einen modellbasierten Steuerungsteil und einen modellbasierten Servoabweichungssteuerungsteil; und
Hinzufügen eines durch den modellbasierten Steuerungsteil erzeugten Drehmoments zu einem durch den modellbasierten Servoabweichungssteuerungsteil erzeugten Drehmoment, um das Steuerungsdrehmoment für das Robotergelenk zu erhalten.

8. Medizinischer Roboter nach Anspruch 7, wobei das vollständige Robotersteuerungsmodell ist:

$$\tau = M(\theta)\ddot{\theta} + V(\theta,\dot{\theta}) + G(\theta) + F(\theta,\dot{\theta})$$

wobei $M(\theta)$ ein Modellparameter einer Trägheitsmatrix eines Roboterbedienarms ist, $V(\theta,\dot{\theta})$ ein Modellparameter einer Geschwindigkeit eines Roboterbedienarms ist, $G(\theta)$ ein Modellparameter einer Schwerkraft eines Roboterbedienarms ist, $F(\theta,\dot{\theta})$ ein Modellparameter einer Reibung eines Roboterbedienarm ist, $\tau$ ein Steuerungsdrehmoment von $n \times 1$ Robotergelenken ist, $n$ ein Freiheitsgrad eines Roboterbedienarmgelenks ist, und $\theta$ ein tatsächlicher Winkel in dem Robotergelenkraum ist, und

wobei die Formel des von dem modellbasierten Steuerungsteil erzeugten Drehmoments ist:

$$\beta = V(\theta,\dot{\theta}) + G(\theta) + F(\theta,\dot{\theta})$$

wobei die Formel des Drehmoments, das durch den modellbasierten Servoabweichungssteuerungsteil erzeugt wird, ist:

$$\tau' = \ddot{\theta}_d + k_v'\dot{E}$$

wobei $\theta_d$ ein gewünschter Winkel in dem Robotergelenkraum ist, $\ddot{\theta}_d$ eine gewünschte Beschleunigung in dem Robotergelenkraum ist, $E = \theta_d - \theta$ eine Abweichung zwischen dem gewünschten Winkel und dem tatsächlichen Winkel in dem Robotergelenkraum ist, $\dot{E}$ eine Abweichung der gewünschten Geschwindigkeit in dem Robotergelenkraum und der tatsächlichen Geschwindigkeit in dem Gelenkraum des Roboters ist, und $k_v'$ ein Verstärkungsfaktor der Geschwindigkeitsabweichung ist, $\beta$ ein Kompensationsdrehmoment ist, das von dem modellbasierten Teil erzeugt wird.

9. Medizinischer Roboter nach Anspruch 1, wobei die verbotene Region von der empfohlenen Region umgeben ist.

10. Steuerungsverfahren eines medizinischen Roboters nach Anspruch 1, umfassend:

Erfassen einer Bedienerbedienkraft;
Erhalten einer Roboterbedienkraft gemäß einem Gefahrenniveau einer Region, in dem sich eine Roboterspitze befindet und gemäß der Beschränkungsstrategie von Anspruch 1, wobei die Regionen wie in Anspruch 1 definiert sind;
Bestimmen einer gewünschten Geschwindigkeit der Roboterspitze gemäß der Bedienerbedienkraft und der Roboterbedienkraft und Umwandeln von Informationen der Geschwindigkeit in eine Steuerungsanweisung; und
Antreiben der Roboterspitze, sich entsprechend der Steuerungsanweisung zu bewegen.

11. Steuerungsverfahren nach Anspruch 10, wobei
der Schritt des Erfassens einer Bedienerbedienkraft umfasst:

Erfassen, durch einen mehrdimensionalen Kraftsensor, der an der Roboterspitze angeordnet ist, einer Bedienerbedienkraft in einem mehrdimensionalen Sensorkoordinatensystem; und
Umwandeln der Bedienerbedienkraft in dem mehrdimensionalen Sensorkoordinatensystem in eine Bedienerbedienkraft in dem kartesischen Raum durch ein Bedienerbedienkraftberechnungsmodul gemäß einem Roboterkraftsteuerungsmodell, oder
wobei der Schritt des Erfassens einer Bedienerbedienkraft umfasst:
Detektieren eines Gelenkdrehmoments des Roboters; und
Entkoppeln des Gelenkdrehmoments des Roboters durch Dynamik, um die Bedienerbedienkraft zu erhalten.

12. Steuerungsverfahren nach Anspruch 10, wobei
der Schritt des Erhaltens einer Roboterbedienkraft gemäß dem Gefahrenniveau einer Region, in der sich eine Roboterspitze befindet, umfasst:

Erstellen eines medizinischen Objektmodells;
Kategorisieren des medizinischen Objektmodells in Regionen und Einstellen unterschiedlicher Roboterbedienkräfte für unterschiedliche Regionen;
Anpassen des medizinischen Objektmodells an Koordinaten der Roboterspitze; und
Erfassen von Positionskoordinaten der Roboterspitze und Bestimmen der Region des medizinischen Objektmodells, in dem sich die Roboterspitze befindet, um eine entsprechende Roboterbedienkraft zu erhalten.

**13.** Steuerungsverfahren nach Anspruch 10, wobei
der Schritt des Bestimmens einer Geschwindigkeit der Roboterspitze gemäß der Bedienerbedienkraft und der Roboterbedienkraft zusammen umfasst:

Erhalten einer gewünschten Geschwindigkeit der Roboterspitze in dem kartesischen Raum gemäß der Bedienerbedienkraft und der Roboterbedienkraft;
Umwandeln der gewünschten Geschwindigkeit der Roboterspitze in den kartesischen Raum, um eine gewünschte Geschwindigkeit in einem Robotergelenkraum gemäß einer Jacobi-Transformation zu erhalten; und
Entkoppeln der gewünschten Geschwindigkeit in dem Robotergelenkraum unter Verwendung des linearen Entkopplungssteuerungsalgorithmus, um ein Steuerungsdrehmoment eines Robotergelenks zu erhalten.

**Revendications**

**1.** Robot médical comprenant :

une unité d'acquisition de force d'actionnement d'opérateur, configurée pour acquérir une force d'actionnement d'opérateur ;
une unité d'acquisition de force d'actionnement de robot, configurée pour acquérir une force d'actionnement de robot selon un niveau de danger d'une région dans laquelle se trouve une pointe de robot, et comprenant :
un module de génération d'objet médical configuré pour établir un modèle d'objet médical et catégoriser le modèle d'objet médical en différentes régions selon le niveau de danger ; un module de mise en correspondance de coordonnées, configuré pour acquérir les coordonnées de la pointe de robot, mettre en correspondance le modèle d'objet médical avec les coordonnées de la pointe de robot, et déterminer la région du modèle d'objet médical dans laquelle se trouve la pointe de robot ; un module de stratégie de contrainte régionale, configuré pour générer différentes stratégies de contrainte selon différents niveaux de danger des régions dans lesquelles peut se trouver la pointe de robot selon un algorithme de restriction de sécurité ; et un module de calcul de force d'actionnement de robot, configuré pour combiner le module de mise en correspondance de coordonnées et le module de stratégie de contrainte régionale afin d'obtenir une force d'actionnement de robot correspondante ;
un module de commande, configuré pour déterminer une vitesse souhaitée de la pointe de robot selon la force d'actionnement d'opérateur et la force d'actionnement de robot, et convertir des informations de vitesse en une instruction de commande ;
un module d'entraînement ; et
la pointe de robot ;
dans lequel le module d'entraînement est configuré pour entraîner le déplacement de la pointe de robot selon une instruction de commande reçue,
**caractérisé en ce que** des régions du modèle d'objet médical catégorisées selon différents niveaux de danger comprennent une région recommandée, une région sûre et une région interdite ;
également **caractérisé en ce que** la stratégie de contrainte comprend :

l'absence de génération d'une force d'actionnement de robot dans la région recommandée ;
la génération d'une force d'actionnement de robot sous la forme d'une force d'amortissement opposée à la force d'actionnement d'opérateur dans la région sûre afin de réduire une vitesse de la pointe de robot ; et
la génération d'une force d'actionnement de robot à l'écart de la région interdite dans la région interdite.

**2.** Robot médical selon la revendication 1, dans lequel l'unité d'acquisition de force d'actionnement d'opérateur comprend :

un capteur de force multidimensionnel, configuré pour acquérir une force d'entrée d'un opérateur ; et
un module de calcul de force d'actionnement d'opérateur connecté en communication au capteur de force multidimensionnel et configuré pour convertir la force d'entrée acquise par le capteur de force multidimensionnel en force d'actionnement d'opérateur dans un espace cartésien selon un modèle de mappage de force de robot, ou
l'unité d'acquisition de force d'actionnement d'opérateur comprend :

un module de détection de couple d'articulation de robot, configuré pour détecter un couple d'articulation du robot ; et

un module de calcul de force d'actionnement d'opérateur connecté en communication au module de détection de couple d'articulation de robot, le module de calcul de force d'actionnement d'opérateur découplant le couple d'articulation du robot par le biais de la dynamique afin d'obtenir la force d'actionnement d'opérateur.

3. Robot médical selon la revendication 1 ou 2, comprenant en outre un module de filtrage, configuré pour filtrer la force d'actionnement d'opérateur afin d'éliminer par filtrage une gigue survenue pendant un actionnement d'opérateur.

4. Robot médical selon l'une quelconque des revendications 1 à 3, dans lequel le module de commande est configuré pour : obtenir une vitesse souhaitée de la pointe de robot dans l'espace cartésien selon la force d'actionnement d'opérateur et la force d'actionnement de robot ;

convertir la vitesse souhaitée de la pointe de robot dans l'espace cartésien en une vitesse souhaitée de la pointe de robot dans un espace d'articulation de robot selon une transformation jacobienne ; et
découpler la vitesse souhaitée de la pointe de robot dans l'espace d'articulation de robot en utilisant un algorithme de commande de découplage linéaire pour obtenir un couple de commande d'une articulation de robot afin d'obtenir une commande de vitesse du robot.

5. Robot médical selon la revendication 4, dans lequel une formule pour obtenir la vitesse souhaitée de la pointe de robot dans l'espace cartésien par le module de commande selon la force d'actionnement d'opérateur et la force d'actionnement de robot est :

$$\dot{\vec{X}}_{des} = K_V (\vec{F_h} - \lambda \bullet \vec{F_b})$$

dans laquelle $\vec{F_h}$ est la force d'actionnement d'opérateur, $\vec{F_b}$ est la force d'actionnement de robot, $K_V$ est un coefficient de transformation proportionnelle d'une force de la pointe de robot dans l'espace cartésien à une vitesse de la pointe de robot dans l'espace cartésien, $\dot{\vec{X}}_{des}$ est la vitesse souhaitée de la pointe de robot dans l'espace cartésien, et $\lambda$ est un facteur d'échelle de la force d'actionnement de robot générée en raison d'un dépassement d'une limite.

6. Robot médical selon la revendication 4, dans lequel une formule pour obtenir la vitesse souhaitée dans l'espace d'articulation de robot par le module de commande selon la vitesse souhaitée de la pointe de robot dans l'espace cartésien est :

$$\dot{\theta}_d = J_{kins}^{-1} \bullet \dot{\vec{X}}_{des}$$

dans laquelle $J_{kins}^{-1}$ est une matrice jacobienne inverse dans le cadre de la configuration actuelle du robot médical, et $\dot{\theta}_d$ est la vitesse souhaitée dans l'espace d'articulation de robot.

7. Robot médical selon la revendication 4, dans lequel le découplage, par le module de commande, de la vitesse souhaitée dans l'espace d'articulation de robot en utilisant un algorithme de commande de découplage linéaire pour obtenir le couple de commande de l'articulation de robot afin d'obtenir la commande de vitesse du robot comprend :

l'établissement d'un modèle de commande de robot complet ;
la catégorisation du modèle de commande de robot en une partie de commande basée sur un modèle et une partie de servocommande d'écart basée sur un modèle ; et
l'ajout d'un couple généré par la partie de commande basée sur un modèle à un couple généré par la partie de servocommande d'écart basée sur un modèle pour obtenir le couple de commande pour l'articulation de robot.

8. Robot médical selon la revendication 7, dans lequel le modèle de commande de robot complet est :

$$\tau = M(\theta)\ddot{\theta} + V(\theta,\dot{\theta}) + G(\theta) + F(\theta,\dot{\theta})$$

dans lequel $M(\theta)$ est un paramètre de modèle d'une matrice d'inertie d'un bras d'actionnement de robot, $V(\theta, \dot{\theta})$ est un paramètre de modèle d'une vitesse d'un bras d'actionnement de robot, $G(\theta)$ est un paramètre de modèle d'une gravité d'un bras d'actionnement de robot, $F(\theta,\dot{\theta})$ est un paramètre de modèle d'un frottement d'un bras d'actionnement de robot, $\tau$ est un couple de commande de n $\times$ 1 articulations de robot, $n$ est un degré de liberté d'une articulation de bras d'actionnement de robot, et $\theta$ est un angle réel dans l'espace d'articulation de robot, et
dans lequel
la formule du couple généré par la partie de commande basée sur un modèle est :

$$\beta = V(\theta,\dot{\theta}) + G(\theta) + F(\theta,\dot{\theta})$$

la formule du couple généré par la partie de servocommande d'écart basée sur un modèle est :

$$\tau' = \ddot{\theta}_d + k_v' \dot{E}$$

dans laquelle $\theta_d$ est un angle souhaité dans l'espace d'articulation de robot, $\dot{\theta}_d$ est une accélération souhaitée dans l'espace d'articulation de robot, E = $\theta_d$ - $\theta$ est un écart entre l'angle souhaité et l'angle réel dans l'espace d'articulation de robot, $\dot{E}$ est un écart de la vitesse souhaitée dans l'espace d'articulation de robot et la vitesse réelle dans l'espace d'articulation de robot, et $k_v'$ est un facteur de gain de l'écart de vitesse, $\tau'$ est un couple généré par la partie de servocommande d'écart, $\beta$ est un couple de compensation généré par la partie basée sur un modèle.

9. Robot médical selon la revendication 1, dans lequel la région interdite est entourée par la région recommandée.

10. Procédé de commande d'un robot médical selon la revendication 1, comprenant :

l'acquisition d'une force d'actionnement d'opérateur ;
l'obtention d'une force d'actionnement de robot selon un niveau de danger d'une région dans laquelle se trouve une pointe de robot et selon la stratégie de contrainte selon la revendication 1, moyennant quoi les régions sont définies selon la revendication 1 ;
la détermination d'une vitesse souhaitée de la pointe de robot selon la force d'actionnement d'opérateur et la force d'actionnement de robot, et la conversion des informations de la vitesse en une instruction de commande ; et
l'entraînement de la pointe de robot pour qu'elle se déplace selon l'instruction de commande.

11. Procédé de revendication selon la revendication 10, dans lequel

l'étape d'acquisition d'une force d'actionnement d'opérateur comprend :

l'acquisition, par un capteur de force multidimensionnel disposé au niveau de la pointe de robot, d'une force d'actionnement d'opérateur dans un système de coordonnées de capteur multidimensionnel ; et
la conversion, par un module de calcul de force d'actionnement d'opérateur selon un modèle de commande de force de robot, de la force d'actionnement d'opérateur dans le système de coordonnées de capteur multidimensionnel en une force d'actionnement d'opérateur dans l'espace cartésien, ou

l'étape d'acquisition d'une force d'actionnement d'opérateur comprend :

la détection d'un couple d'articulation du robot ; et

le découplage du couple d'articulation du robot par le biais de la dynamique afin d'obtenir la force d'actionnement d'opérateur.

**12.** Procédé selon la revendication 10, dans lequel
l'étape d'obtention d'une force d'actionnement de robot selon le niveau de danger d'une région dans laquelle se trouve une pointe de robot comprend :

l'établissement d'un modèle d'objet médical ;
la catégorisation du modèle d'objet médical en régions, et la définition de différentes forces d'actionnement de robot pour différentes régions ;
la mise en correspondance du modèle d'objet médical avec les coordonnées de la pointe de robot ;
l'acquisition de coordonnées de position de la pointe de robot, et la détermination de la région du modèle d'objet médical dans laquelle se trouve la pointe de robot, afin d'obtenir une force d'actionnement de robot correspondante.

**13.** Procédé de commande selon la revendication 10, dans lequel
l'étape de détermination d'une vitesse de la pointe de robot selon la force d'actionnement d'opérateur et la force d'actionnement de robot comprend :

l'obtention d'une vitesse souhaitée de la pointe de robot dans l'espace cartésien selon la force d'actionnement d'opérateur et la force d'actionnement de robot ;
la conversion de la vitesse souhaitée de la pointe de robot dans l'espace cartésien pour obtenir une vitesse souhaitée dans un espace d'articulation de robot selon une transformation jacobienne ; et
le découplage de la vitesse souhaitée dans l'espace d'articulation de robot en utilisant l'algorithme de commande de découplage linéaire pour obtenir un couple de commande d'une articulation de robot.

FIG. 1

FIG. 2

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104470456 A

- WO 02060653 A2

**Non-patent literature cited in the description**

- **HO et al.** ROBOT ASSISTED KNEE SURGERY. ESTABLISHING A FORCE CONTROL STRATEGY INCORPORATING ACTIVE MOTION CONSTRAINT. *IEEE ENGINEERING IN MEDICINE AND BIOLOGY MAGAZINE,* 01 May 1995, vol. 14 (3), ISSN 0739-5175, 292-300